# EUROPEAN PATENT APPLICATION

(11) **EP 4 428 145 A1**
(43) Date of publication of application: **11.09.2024**
(21) Application number: 22889956.3
(22) Date of filing: 01.11.2022
(51) Int. Cl.: C07K 14/335, C07K 4/04, C07K 7/06, C12N 1/15, C12N 1/19, C12N 1/21, C12N 5/10, C12N 15/11, C12N 15/63, C12P 21/02

(54) **ANTIBACTERIAL PEPTIDE**

(30) Priority: 02.11.2021 JP 2021179551
(71) Applicant: Sumitomo Chemical Company, Limited, Tokyo 103-6020 (JP)
(72) Inventor: KOMORI, Yumi, Osaka-shi, Osaka 554-8558 (JP); KUWAHARA, Hiroshi, Osaka-shi, Osaka 554-8558 (JP); AOKI, Mikio, Osaka-shi, Osaka 554-8558 (JP); MIKATA, Kazuki, Osaka-shi, Osaka 554-8558 (JP)
(74) Representative: Vossius & Partner Patentanwälte Rechtsanwälte mbB
(86) International application number: PCT/JP2022/040886
(87) International publication number: WO 2023/080136

(57) **Abstract**

A polypeptide comprising: (a) an amino acid sequence as set forth in SEQ ID NO: 3, SEQ ID NO: 4, or SEQ ID NO: 6; (b) an amino acid sequence with an identity of 80% or more to the amino acid sequence as set forth in SEQ ID NO: 3, SEQ ID NO: 4, or SEQ ID NO: 6; (c) the amino acid sequence as set forth in SEQ ID NO: 3, SEQ ID NO: 4, or SEQ ID NO: 6 with deletion, substitution, insertion, and/or addition of one or several amino acid residues is provided.

## Description

### TECHNICAL FIELD

The present invention relates to an antimicrobial peptide.

### BACKGROUND ART

Antimicrobial peptides are produced by a wide range of living things and play an important defense role for living things. They act quickly as compared to antibiotics and do not remain in the environment due to degradation into amino acids by digestive enzymes and the like, so antimicrobial peptides are less likely to give rise to resistant bacteria. Antimicrobial peptides are expected to be useful as a means to deal with drug-resistant bacteria, which is a serious social issue to address today.

As an antimicrobial peptide produced by a lactic acid bacterium (Lactococcus lactis ATCC-11454 strain), nisin A is known. Nisin A exhibits high antimicrobial activity against many gram-positive bacteria including heat-resistant spore-forming bacteria, is stable against acid, and is degraded by intestinal digestive enzymes into amino acids, so it has been used as a food preservative in practical settings in at least fifty countries. However, nisin A is unstable within the neutral range and the alkaline range, and not highly thermostable within the neutral range. For these reasons, nisin A is only used in food preservatives and the like which are often sold with the pH adjusted to the acidic range. Moreover, because nisin A contains abnormal amino acids resulting from post-translational modification in cells and also has a crosslink structure based on monosulfide bonding of lanthionine and/or the like, it is difficult to synthesize it chemically or by in vitro translation. U.S. Patent No. 4584199 specification (PTL 1), U.S. Patent No. 3295989 specification (PTL 2), International Patent Laying-Open No. WO 89/12399 (PTL 3), and International Patent Laying-Open No. WO 2004/029082 (PTL 4) disclose use of nisin for food preservation.

Polylysine is a naturally-occurring peptide-based antimicrobial substance produced by actinomycetes culture. Polylysine is also used as a food preservative. Polylysine is thermally stable, but its antimicrobial activity is lost at pH9 or higher. It is known that polylysine tends to be adsorbed on food ingredients and lose its antimicrobial activity.

### CITATION LIST

### PATENT LITERATURE

PTL 1: U.S. Patent No. 4584199 specification
PTL 2: U.S. Patent No. 3295989 specification
PTL 3: International Patent Laying-Open No. WO 89/12399
PTL 4: International Patent Laying-Open No. WO 2004/029082

### SUMMARY OF INVENTION

### TECHNICAL PROBLEM

An object of the present invention is to provide a new polypeptide having antimicrobial activity.

### SOLUTION TO PROBLEM

The present invention relates to the following items.
[1] A polypeptide with antimicrobial activity, comprising any one of the following amino acid sequences:
   (1a) an amino acid sequence as set forth in SEQ ID NO: 3;
   (1b) an amino acid sequence with an identity of 80% or more to the amino acid sequence as set forth in SEQ ID NO: 3;
   (1c) the amino acid sequence as set forth in SEQ ID NO: 3 with deletion, substitution, insertion, and/or addition of one or several amino acid residues;
   (1d) the amino acid sequence of any one of (1a) to (1c) with deletion of 1 to 26 amino acid residues from a C terminus thereof;
   (2a) an amino acid sequence as set forth in SEQ ID NO: 4;
   (2b) an amino acid sequence with an identity of 80% or more to the amino acid sequence as set forth in SEQ ID NO: 4;
   (2c) the amino acid sequence as set forth in SEQ ID NO: 4 with deletion, substitution, insertion, and/or addition of one or several amino acid residues;
   (2d) the amino acid sequence of any one of (2a) to (2c) with deletion of 1 to 8 amino acid residues from a C terminus thereof;
   (3a) an amino acid sequence as set forth in SEQ ID NO: 6;
   (3b) an amino acid sequence with an identity of 80% or more to the amino acid sequence as set forth in SEQ ID NO: 6; and
   (3c) the amino acid sequence as set forth in SEQ ID NO: 6 with deletion, substitution, insertion, and/or addition of one or several amino acid residues.
[2] A polypeptide with antimicrobial activity, comprising at least a partial amino acid sequence of the amino acid sequence of any one of (1a) to (1c) according to [1]
[3] A precursor polypeptide for the polypeptide according to [1] or [2].
[4] The polypeptide according to [3], having an amino acid sequence as set forth in SEQ ID NO: 1.
[5] A nucleic acid coding for the polypeptide according to any one of [1] to [4].
[6] The nucleic acid according to [5], having a DNA sequence as set forth in SEQ ID NO: 2.
[7] A vector comprising the nucleic acid according to [5] or [6].
[8] A transformant in which the nucleic acid according to [5] or [6] is introduced.
[9] A method of producing a polypeptide, comprising using the transformant according to [8].
[10] A composition comprising the polypeptide according to [1] or [2] and an additive.

### ADVANTAGEOUS EFFECTS OF INVENTION

The present invention makes it possible to provide a new polypeptide having antimicrobial activity.

### BRIEF DESCRIPTION OF DRAWINGS

Fig. 1 is a view of the structure of a novel polypeptide, lactobacin A.
Fig. 2 gives a result of homology analysis performed in Experiment 4 between the amino acid sequence of lactobacin A and the amino acid sequence of lactococcin A.
Fig. 3 gives a result of homology analysis performed in Experiment 4 between the amino acid sequence of lactobacin A and the amino acid sequence of a hypothetical protein derived from Lactobacillus johnsonii.
Fig. 4 gives results of antimicrobial activity testing of a full-length polypeptide and a partial polypeptide of lactobacin A performed in Experiment 5.
Fig. 5 gives results of pH-stability testing of a full-length polypeptide of lactobacin A performed in Experiment 6.
Fig. 6 gives results of pH-stability testing of a partial polypeptide of lactobacin A performed in Experiment 6.
Fig. 7 gives results of thermal stability testing of a full-length polypeptide and a partial polypeptide of lactobacin A performed in Experiment 7.
Fig. 8 gives results of oxidation stability testing of a full-length polypeptide of lactobacin A performed in Experiment 8.

### DESCRIPTION OF EMBODIMENTS

In the following, a detailed description will be given of embodiments of the present invention, but the scope of the present invention is not limited to the below embodiments.

### [Polypeptide with Antimicrobial Activity]

A polypeptide according to an embodiment of the present invention includes any one of the following amino acid sequences. Preferably, the polypeptide has antimicrobial activity.
(1a) An amino acid sequence as set forth in SEQ ID NO: 3,
(1b) an amino acid sequence with an identity of 80% or more to the amino acid sequence as set forth in SEQ ID NO: 3,
(1c) the amino acid sequence as set forth in SEQ ID NO: 3 with deletion, substitution, insertion, and/or addition of one or several amino acid residues, and
(1d) the amino acid sequence of any one of (1a) to (1c) with deletion of 1 to 26 amino acid residues from a C terminus thereof.

The polypeptide according to an embodiment of the present invention may be a polypeptide that includes an amino acid sequence with an identity of, for example, 85% or more, or 90% or more, or 95% or more, or 96% or more, or 97% or more, or 98% or more, or 99% or more to the amino acid sequence as set forth in SEQ ID NO: 3. The polypeptide according to an embodiment of the present invention may consist of the amino acid sequence as set forth in SEQ ID NO: 3.

"One or several" varies depending on the position, type, and the like of the amino acid residue(s) in the conformation of the protein, but it may be from 1 to 10, for example. The polypeptide according to an embodiment of the present invention may be a polypeptide that includes the amino acid sequence as set forth in SEQ ID NO: 3 with deletion, substitution, insertion, and/or addition of not less than 1 and not more than 10 amino acid residues, or not more than 9, or not more than 8, or not more than 7, or not more than 6, or not more than 5, or not more than 4, or not more than 3, or not more than 2, for example.

An example of the substitution, deletion, insertion, and/or addition of amino acid residues is a conservative variation in which the function of the polypeptide is properly retained. A typical conservative variation is conservative substitution. Conservative substitution refers to a variation in which substitution takes place between Phe, Trp, and Tyr in the case where an aromatic amino acid is at the moiety for substitution, or between Leu, Ile, and Val in the case where a hydrophobic amino acid is at the moiety for substitution, or between Gln and Asn in the case of a polar amino acid, or between Lys, Arg, and His in the case of a basic amino acid, or between Asp and Glu in the case of an acidic amino acid, or between Ser and Thr in the case of a hydroxy-containing amino acid. Examples of the substitution that is regarded as conservative substitution include substitution from Ala to Ser or Thr, substitution from Arg to Gln, His, or Lys, substitution from Asn to Glu, Gln, Lys, His, or Asp, substitution from Asp to Asn, Glu, or Gln, substitution from Cys to Ser or Ala, substitution from Gln to Asn, Glu, Lys, His, Asp, or Arg, substitution from Glu to Gly, Asn, Gln, Lys, or Asp, substitution from Gly to Pro, substitution from His to Asn, Lys, Gln, Arg, or Tyr, substitution from Ile to Leu, Met, Val, or Phe, substitution from Leu to Ile, Met, Val, or Phe, substitution from Lys to Asn, Glu, Gln, His, or Arg, substitution from Met to Ile, Leu, Val, or Phe, substitution from Phe to Trp, Tyr, Met, Ile, or Leu, substitution from Ser to Thr or Ala, substitution from Thr to Ser or Ala, substitution from Trp to Phe or Tyr, substitution from Tyr to His, Phe, or Trp, and substitution from Val to Met, Ile, or Leu.

The polypeptide according to an embodiment of the present invention may be a polypeptide that includes at least a partial amino acid sequence of the amino acid sequence of any one of (1a) to (1c). Preferably, the polypeptide has antimicrobial activity. The polypeptide that includes at least a partial amino acid sequence of the amino acid sequence of any one of (1a) to (1c) may consist of the amino acid sequence of the corresponding one of (1a) to (1c) with deletion of amino acid residue(s) from the C terminus and/or the N terminus thereof, or may consist of the amino acid sequence with deletion of not less than 1 and not more than 26 amino acid residues from the C terminus thereof, or not more than 20, or not more than 15, or not more than 10, or not more than 9, or not more than 8, or not more than 7, or not more than 6, or not more than 5, or not more than 4, or not more than 3, or not more than 2, or may consist of the amino acid sequence with deletion of not less than 1 and not more than 10 amino acid residues from the N terminus thereof, or not more than 9, or not more than 8, or not more than 7, or not more than 6, or not more than 5, or not more than 4, or not more than 3, or not more than 2. The polypeptide that includes at least a partial amino acid sequence of the amino acid sequence of any one of (1a) to (1c) may be the amino acid sequence of the corresponding one of (1a) to (1c) without deletion of amino acid residue(s) from the C terminus or the N terminus thereof. The polypeptide that includes at least a partial amino acid sequence of the amino acid sequence of any one of (1a) to (1c) may be a polypeptide consisting of the amino acid sequence of any one of (2a) to (2d) described below, or may be a polypeptide consisting of the amino acid sequence of any one of (3a) to (3c).

The amino acid sequence as set forth in SEQ ID NO: 3 is the amino acid sequence of a mature protein predicted from the genome sequence of a lactic acid bacterium named Lactobacillus sp. SC-2001. Also, a novel polypeptide consisting of the amino acid sequence as set forth in SEQ ID NO: 3 is called lactobacin A.

Lactobacin A has antimicrobial activity against a wide range of bacteria species, and, in particular, has antimicrobial activity against bacteria that cause food poisoning, such as heat-resistant spore-forming bacteria, Staphylococcus aureus, and Streptococcus pneumoniae. Antimicrobial activity of the polypeptide can be measured by an antimicrobial activity test method described below, for example. Lactobacin A is highly pH-stable, and has antimicrobial activity not only within the acidic range (pH4.0, for example) but also within the neutral range (pH7.4, for example) and within the alkaline range (pH10.0, for example). Lactobacin A is highly thermostable, and, for example, it has antimicrobial activity after heat treatment (at temperatures of 80°C, 100°C, 121°C, and the like) within the neutral range. Lactobacin A is highly oxidation-stable, and, for example, it has antimicrobial activity after treatment with 10-mM hydrogen peroxide. The antimicrobial activity of lactobacin A is equal to or higher than that of polylysine. Thus, lactobacin A is a naturally-occurring polypeptide with high antimicrobial activity, and due to its wide applicable pH range and its stability against heat, oxidation, and the like, it is widely applicable as an antimicrobial component. Because lactobacin A is a linear polypeptide having no specially-modified amino acid residues, chemical synthesis thereof is easy to perform.

A polypeptide according to an embodiment of the present invention includes any one of the following amino acid sequences. Preferably, the polypeptide has antimicrobial activity.
(2a) An amino acid sequence as set forth in SEQ ID NO: 4,
(2b) an amino acid sequence with an identity of 80% or more to the amino acid sequence as set forth in SEQ ID NO: 4,
(2c) the amino acid sequence as set forth in SEQ ID NO: 4 with deletion, substitution, insertion, and/or addition of one or several amino acid residues, and
(2d) the amino acid sequence of any one of (2a) to (2c) with deletion of 1 to 8 amino acid residues from a C terminus thereof.

The polypeptide according to an embodiment of the present invention may be a polypeptide that includes an amino acid sequence with an identity of, for example, 85% or more, or 90% or more, or 95% or more, or 96% or more, or 97% or more, or 98% or more, or 99% or more to the amino acid sequence as set forth in SEQ ID NO: 4. The polypeptide according to an embodiment of the present invention may consist of the amino acid sequence as set forth in SEQ ID NO: 4.

The polypeptide according to an embodiment of the present invention may be a polypeptide that includes the amino acid sequence as set forth in SEQ ID NO: 4 with deletion, substitution, insertion, and/or addition of not less than 1 and not more than 10 amino acid residues, or not more than 9, or not more than 8, or not more than 7, or not more than 6, or not more than 5, or not more than 4, or not more than 3, or not more than 2, for example.

The polypeptide according to an embodiment of the present invention may be a polypeptide that includes at least a partial amino acid sequence of the amino acid sequence of any one of (2a) to (2c). Preferably, the polypeptide has antimicrobial activity. The polypeptide that includes at least a partial amino acid sequence of the amino acid sequence of any one of (2a) to (2c) may consist of the amino acid sequence of the corresponding one of (2a) to (2c) with deletion of amino acid residue(s) from the C terminus and/or the N terminus thereof, or may consist of the amino acid sequence with deletion of not less than 1 and not more than 8 amino acid residues from the C terminus thereof, or not more than 7, or not more than 6, or not more than 5, or not more than 4, or not more than 3, or not more than 2, or may consist of the amino acid sequence with deletion of not less than 1 and not more than 10 amino acid residues from the N terminus thereof, or not more than 9, or not more than 8, or not more than 7, or not more than 6, or not more than 5, or not more than 4, or not more than 3, or not more than 2. The polypeptide that includes at least a partial amino acid sequence of the amino acid sequence of any one of (2a) to (2c) may be the amino acid sequence of the corresponding one of (2a) to (2c) without deletion of amino acid residue(s) from the C terminus or the N terminus thereof. The polypeptide that includes at least a partial amino acid sequence of the amino acid sequence of any one of (2a) to (2c) may be a polypeptide consisting of the amino acid sequence of any one of (3a) to (3c) described below.

A polypeptide according to an embodiment of the present invention includes any one of the following amino acid sequences. Preferably, the polypeptide has antimicrobial activity.
(3a) An amino acid sequence as set forth in SEQ ID NO: 6,
(3b) an amino acid sequence with an identity of 80% or more to the amino acid sequence as set forth in SEQ ID NO: 6, and
(3c) the amino acid sequence as set forth in SEQ ID NO: 6 with deletion, substitution, insertion, and/or addition of one or several amino acid residues.

The polypeptide according to an embodiment of the present invention may be a polypeptide that includes an amino acid sequence with an identity of, for example, 85% or more, or 90% or more, or 95% or more, or 96% or more, or 97% or more, or 98% or more, or 99% or more to the amino acid sequence as set forth in SEQ ID NO: 6. The polypeptide according to an embodiment of the present invention may consist of the amino acid sequence as set forth in SEQ ID NO: 6.

The polypeptide according to an embodiment of the present invention may be a polypeptide that includes the amino acid sequence as set forth in SEQ ID NO: 6 with deletion, substitution, insertion, and/or addition of not less than 1 and not more than 5 amino acid residues, or not more than 4, or not more than 3, or not more than 2, for example.

The amino acid sequence as set forth in SEQ ID NO: 4 is an amino acid sequence from position 1 to 17 of the amino acid sequence as set forth in SEQ ID NO: 3. The amino acid sequence as set forth in SEQ ID NO: 6 is an amino acid sequence from position 1 to 9 of the amino acid sequence as set forth in SEQ ID NO: 3. A polypeptide consisting of the amino acid sequence as set forth in SEQ ID NO: 4 and a polypeptide consisting of the amino acid sequence as set forth in SEQ ID NO: 6 both lack a C-terminal amino acid residue(s) of lactobacin A, but have antimicrobial activity. A polypeptide having a short amino acid sequence is easy to chemically synthesize than a polypeptide having a long amino acid sequence. A polypeptide that has at least part of lactobacin A, even when it is a polypeptide that lacks a C-terminal amino acid residue(s) of lactobacin A, for example, has pH stability, thermal stability, and oxidation stability similarly to lactobacin A.

A polypeptide according to an embodiment of the present invention may include a signal peptide, and may be a precursor polypeptide for the above-mentioned polypeptide. The precursor polypeptide can be cleaved by peptidase inside of cells or outside of cells to become the above-mentioned polypeptide. Usually, the precursor polypeptide includes an amino acid sequence that is to be cleaved by a particular peptidase. Examples of the precursor polypeptide include a polypeptide having an amino acid sequence as set forth in SEQ ID NO: 1. For example, a precursor polypeptide synthesized as SEQ ID NO: 1 in cells is cleaved on the cell membrane to become mature lactobacin A that has antimicrobial activity. The polypeptide according to an embodiment of the present invention may be a secretory polypeptide, or may be a membrane-bound polypeptide.

An embodiment of the present invention is a composition that includes the above-mentioned polypeptide. An embodiment of the present invention may be a bacterial-growth inhibitor agent or a bactericide that includes the above-mentioned polypeptide. An embodiment of the present invention is a bacterial-growth inhibitory or bactericidal method with the use of the above-mentioned polypeptide. The composition may further include an additive. Examples of the additive include a surfactant, a preserving agent, a preservative, a pH regulator, a thickener, an excipient, a colorant, and a fragrance, which may be included in combination.

The surfactant may be an anionic surfactant, a nonionic surfactant, or an amphoteric surfactant. Examples of the anionic surfactant include N-acylamino acid salts, α-olefin sulfonic acid salts, N-acylsulfonic acid salts, alkyl sulfuric acid salts (such as sodium lauryl sulfate, for example), glycerol fatty acid ester sulfuric acid salts, and the like. Examples of the nonionic surfactant include polyoxyethylene alkyl ether, polyoxyethylene-polyoxypropylene block copolymer, polyoxyethylene hydrogenated castor oil, glyceryl ester polyoxyethylene ether, alkylol amide, sucrose fatty acid ester, glycerol fatty acid ester, sorbitan fatty acid ester, propylene glycol fatty acid ester, glycerol organic acid fatty acid ester, polyglycerol fatty acid ester, calcium stearoyl lactylate, sodium stearoyl lactylate, polyoxyethylene sorbitan fatty acid ester, alkyl glycoside, and the like. Examples of the amphoteric surfactant include alkyl betaine-based surfactants, amine oxide-based surfactants, imidazolinium betaine-based surfactants. Specific examples thereof include 2-alkyl-N-carboxymethyl-N-hydroxyethyl imidazolinium betaine, lauryl dimethylaminoacetic acid betaine, coconut alkyl betaine (coconut alkyl dimethylaminoacetic acid betaine), stearyldimethylaminoacetic acid betaine, sodium stearyl dimethyl betaine, cocamide alkyl betaine, palm acid amidopropyl betaine, lauryl amidopropyl betaine, ricinoleic acid amidopropyl betaine, stearyl dihydroxyethyl betaine, and the like.

Examples of the preserving agent or the preservative include sodium benzoate, p-hydroxybenzoic acid esters such as methylparaben, ethylparaben, butylparaben, isopropylparaben, propylparaben, isobutylparaben, and benzylparaben, alcohols such as phenoxyethanol and ethanol, sorbic acid, benzoic acid, dehydroacetic acid, propionic acid, and salts thereof, salts such as sodium chloride, ethylenediaminetetraacetate, benzalkonium chloride, benzethonium chloride, cetylpyridinium chloride, alkyl diaminoethyl glycine hydrochloride, and the like.

Examples of the pH regulator include acids and alkalis such as acetic acid, hydrochloric acid, sulfuric acid, nitric acid, citric acid, phosphoric acid, malic acid, gluconic acid, maleic acid, succinic acid, glutamic acid, pyrophosphoric acid, tartaric acid, acetic acid sodium hydroxide, potassium hydroxide, sodium hydroxide, sodium acetate, sodium carbonate, potassium carbonate, sodium citrate, sodium hydrogen citrate, phosphoric acid, sodium phosphate, sodium monohydrogen phosphate, sodium dihydrogen phosphate, and potassium dihydrogen phosphate, buffers, and the like.

Examples of the thickener include methylcellulose, hydroxyethylcellulose, hydroxypropylcellulose, carboxymethylcellulose, and salts thereof, organic bonding agents such as pullulan, gelatin, carrageenan, sodium alginate, xanthan gum, sodium polyacrylate, gum arabic, guar gum, locust bean gum, polyvinyl alcohol, polyvinylpyrrolidone, and carboxyvinyl polymer, thickening silicic anhydride, inorganic bonding agents such as bentonite, and the like.

Examples of the excipient include crystalline cellulose, powder cellulose, potato starch, corn starch, light silicic anhydride, hydrous silicon dioxide, silicon dioxide, precipitated calcium carbonate, anhydrous calcium hydrogen phosphate, magnesium oxide, calcium lactate, calcium silicate, magnesium aluminometasilicate, synthesized hydrotalcite, synthesized aluminum silicate, lactose, white sugar, D-mannitol, erythritol, glucose, fructose, and the like.

### [Nucleic Acid Coding for Polypeptide]

A nucleic acid according to an embodiment of the present invention is a nucleic acid coding for the above-mentioned polypeptide. The nucleic acid may be DNA or RNA. The nucleic acid may or may not include a start codon at the 5' end side thereof and a stop codon at the 3' end side thereof. The nucleic acid may or may not include an intron sequence. An example of the nucleic acid according to an embodiment of the present invention has a DNA sequence as set forth in SEQ ID NO: 2. SEQ ID NO: 2 is a sequence derived from the genome sequence of a lactic acid bacterium named Lactobacillus sp. SC-2001, and codes for the precursor polypeptide as set forth in SEQ ID NO: 1.

The nucleic acid is not limited to the nucleic acid sequence of Lactobacillus sp. SC-2001, and may be a nucleic acid that includes a base sequence in which a codon coding for an amino acid in a coding region is substituted by another codon coding for the same amino acid. The nucleic acid according to an embodiment of the present invention may be a nucleic acid that includes a base sequence in which the codon usage is changed for the purpose of enhancing expression of the polypeptide according to the present invention.

For example, the nucleic acid according to the present invention can be obtained by chemical synthesis, or by PCR using the genome sequence of Lactobacillus sp. SC-2001 as a template.

### [Vector]

A vector according to an embodiment of the present invention includes the above-mentioned nucleic acid. The vector is a nucleic acid molecule that is capable of amplifying and retaining DNA, and examples thereof include expression vectors and cloning vectors. In one example, the above-mentioned nucleic acid is inserted into an expression vector, introduced into a host cell and the like, and expresses a polypeptide with antimicrobial activity. The vector, introduced in a host cell, is capable of expressing a polypeptide in the host cell. The expression vector may have a promoter sequence and a terminator sequence for expression of a gene it harbors. The vector can be constructed by introducing the above-mentioned nucleic acid into a basic vector in accordance with an ordinary genetic engineering technique. The basic vector may have a selection marker sequence.

For example, the vector may be a bacterial plasmid-derived vector, a yeast plasmid-derived vector, a viral vector, a cosmid vector, a phagemid vector, an artificial chromosome vector, and/or the like. Examples of the basic vector include pBR322, pUC plasmid vector, pET-based plasmid vector, and the like. Specifically, in the case where Escherichia coli is used as the host cell, pUC19, pUC18, pUC119, pBluescriptII, pET32, and/or the like may be used. In the case where a mammalian cell is used as the host cell, pRc/RSV, pRc/CMV, a retroviral vector, an adenoviral vector, an adeno-associated virus vector, and/or the like may be used, for example.

### [Transformant]

A transformant according to an embodiment of the present invention is a cell in which the above-mentioned nucleic acid is introduced. The nucleic acid may be harbored by the vector, when it is introduced into the host cell. The transformant is capable of expressing the polypeptide according to the present invention.

The cell into which the nucleic acid is to be introduced may be a eukaryotic cell or a prokaryotic cell, and examples thereof include bacteria, fungi, plant cells, animal cells, and insect cells. The cell may be a cell of yeast, Escherichia coli, or a mammal, and the mammal may be a human, a cow, a horse, sheep, a monkey and an ape, a pig, a mouse, a rat, a hamster, a guinea pig, a rabbit, a dog, and/or the like.

The method for introducing the nucleic acid into the cell may be a chemical technique such as a calcium phosphate method, a DEAE-dextran method, and a cationic liposome method; a biological technique such as an adenoviral vector, a vaccinia virus vector, a retroviral vector, and an HVJ liposome; a physical technique such as electroporation, DNA direct injection, and gene gun; and/or the like. An introduction method that is suitable for the introduction-target cell can be selected.

In the host cell, the nucleic acid and the vector may be retained outside the chromosome, or may be harbored inside the chromosome. The nucleic acid and the vector are retained in the transformant, preferably in a state where they are capable of expressing the gene under the control of a promoter that functions in the host cell.

The transformant in which the nucleic acid is introduced may be selected with the use of a selection marker. For example, a selection marker gene is introduced into the host cell simultaneously with the nucleic acid according to an embodiment of the present invention, and cultured by a method suitable for the characteristics of the selection marker. In the case where the selection marker gene is a gene capable of providing drug resistance against a selective agent that has activity to kill the host cell, the host cell in which the nucleic acid is introduced can be cultured in a medium that contains the selective agent.

### [Method of Producing Polypeptide]

The polypeptide according to the present invention can be produced by chemical synthesis, or by using the transformant in which the above-mentioned nucleic acid is introduced. The polypeptide according to the present invention can be obtained by culturing the transformant in which the above-mentioned nucleic acid is introduced, in a proper medium. The culturing of the transformant may be carried out by the method for culturing the host cell. In the case where the transformant is a microorganism, the culturing can be carried out by using various types of media containing, as appropriate, carbon sources, nitrogen sources, organic and inorganic salts, and/or the like that are usually used in microbial culture, for example.

As a result of culturing the transformant, a culture that contains the polypeptide according to the present invention is obtained. For example, the polypeptide according to the present invention may be accumulated inside the transformant and/or outside the transformant (for example, in the culture supernatant of the transformant). The polypeptide according to the present invention can be obtained from the transformant and/or from the culture supernatant thereof. The polypeptide according to the present invention may be a polypeptide obtained by disruption, lysis, extraction, and purification of the transformant as appropriate. The disruption, lysis, extraction, and the like can be carried out by known methods. Examples of the methods to do these operations include an ultrasonic disruption method, a dyno-mill method, beads disruption, french press disruption, and lysozyme treatment. One of these methods may be used alone, or two or more of these may be used in combination as appropriate. The purification can be carried out by a known method that is used for polypeptide purification. Examples of the method include ammonium sulfate fractionation, ionexchange chromatography, hydrophobic chromatography, affinity chromatography, gel-filtration chromatography, and isoelectric precipitation. One of these methods may be used alone, or two or more of these may be used in combination as appropriate. The transformant may be collected from the culture by centrifugation and/or the like. In the case where the polypeptide according to the present invention is accumulated in the culture supernatant, the culture supernatant can be obtained by centrifugation and/or the like, and the polypeptide according to the present invention can be collected from the culture supernatant. The polypeptide according to the present invention may be a fraction of the culture supernatant of the transformant. Alternatively, the polypeptide according to the present invention can be obtained by culturing Lactobacillus sp. SC-2001 in a proper medium such as MRS liquid medium. The polypeptide according to the present invention may be collected, extracted, and purified from the culture of Lactobacillus sp. SC-2001 by the same method as used in the transformant culturing.

### [Examples]

In the following, a more detailed description will be given of the present invention by way of Examples, but these Examples are not intended to limit the scope of the present invention.

### [Experiment 1. Antimicrobial Activity Test of Culture Supernatant of Lactobacillus sp. SC-2001]

The presence or absence of antimicrobial activity in culture supernatant of Lactobacillus sp. SC-2001 was investigated. As a control, culture supernatant of a nisin A-producing strain named Lactococcus lactis ATCC-11454 was used. The culture supernatant of each of Lactobacillus sp. SC-2001 and the nisin A-producing strain was a supernatant obtained by culturing the bacteria in MRS Broth at a temperature of 30°C and then making the bacterial cells precipitated by centrifugation.

### (Experiment 1A)

Investigation was carried out to see if culture supernatant of Lactobacillus sp. SC-2001 could inhibit the growth of Ralstonia solanacearum, Staphylococcus aureus, Lactobacillus sakei, and Listeria innocua. Antimicrobial activity was evaluated by a spot-on-lawn method that involved spotting 10 µL of culture supernatant of Lactobacillus sp. SC-2001 on the plate-cultured test bacteria. The details of the spot-on-lawn method are as described by Mayr-Harting, A. et al., Methods Microbiol., 7A, 315-422(1972). Firstly, the strain specified in Table 1 was precultured overnight. The test bacteria were mixed and diluted with a soft agar medium so as to make the absorbance at a measurement wavelength of 600 nM (OD600) fall within the range of 0.1 to 0.3, and the resultant was spread over an agar medium to prepare a measurement medium specified in Table 1. Culture supernatant of Lactobacillus sp. SC-2001 or the nisin A-producing strain was spotted on the test bacteria, and incubated overnight at the temperature specified in Table 1. The size of the growth inhibition circle thus formed was evaluated as the antimicrobial activity. Results are shown in Table 2.

### (Experiment 1B)

Investigation was carried out to see if culture supernatant of Lactobacillus sp. SC-2001 could inhibit the growth of Bacillus subtilis, Bacillus cereus, and Streptococcus pneumoniae. The strain specified in Table 2 was precultured overnight. The test bacteria were suspended in sterilized physiological saline, and adjustment was made to achieve McFarland No. 0.5 (about 1 to 2×10⁸ cfu/mL). The resultant was further 10-fold diluted, and thereby a test bacteria suspension of about 1 to 2×10⁷ cfu/mL was obtained. The resulting test bacteria suspension in an amount of 100 µL was added in drops to a measurement medium, and spread evenly with a bacteria spreader. Once the surface of the agar medium dried, 10 µL of culture supernatant of Lactobacillus sp. SC-2001 or the nisin A-producing strain was added in drops, and cultured at 35°C for 18 to 24 hours in an aerobic environment. The size of the growth inhibition circle thus formed was evaluated as the antimicrobial activity. Results are shown in Table 2.

**[Table 1]**

| Experiment 1A | | | | | |
|---|---|---|---|---|---|
| Test bacteria | Accession institution No. | Preculture medium | Measurement medium | Culture conditions | Temperature |
| *Ralstonia solanacearum* | MAFF211534 | LB medium | Double-layer medium made of LB soft agar containing mixed/diluted test bacteria over LB agar medium | Aerobic culturing | 30°C |
| *Staphylococcus aureus* | ATCC 12600 | NB medium | Double-layer medium made of NB soft agar containing mixed/diluted test bacteria over NB agar medium | Aerobic culturing | 37°C |
| *Lactobacillus sakei* | NBRC15893 | MRS medium | Double-layer medium made of Lactobacilli AOAC agar containing mixed/diluted test bacteria over MRS agar medium | Aerobic culturing | 30°C |
| *Listeria innocua* | ATCC33090 | BHI medium | Double-layer medium made of BHI soft agar containing mixed/diluted test bacteria over BHI agar medium | Aerobic culturing | 37°C |

| Experiment 1B | | | | | |
|---|---|---|---|---|---|
| Test bacteria | Accession institution No. | Preculture medium | Measurement medium | Culture conditions | Temperature |
| *Bacillus subtilis* | NBRC13719 | Mueller-Hinton II agar medium | Mueller-Hinton II agar medium | Aerobic culturing | 35°C |
| *Bacillus cereus* | ATCC14579 | Mueller-Hinton II agar medium | Mueller-Hinton II agar medium | Aerobic culturing | 35°C |
| *Streptococcus pneumoniae* | ATCC49619 | Mueller-Hinton agar medium with 5% sheep blood | Mueller-Hinton agar medium with 5% sheep blood | 5% CO₂ culturing | 35°C |

**[Table 2]**

| Test bacteria | | *Lactobacillus sp.* SC-2001 | Nisin A-producing strain |
|---|---|---|---|
| *Ralstonia solanacearum* | Bacteria causing bacterial wilt | + | - |
| *Staphylococcus aureus* | Bacteria causing spoilage/food poisoning | + | ++ |
| *Bacillus subtilis* | Bacteria causing spoilage/food poisoning | + | ++ |
| *Bacillus cereus* | Bacteria causing spoilage/food poisoning | + | ++ |
| *Lactobacillus sakei* | Bacteria causing spoilage/food poisoning | ++ | ++ |
| *Listeria innocua* | Bacteria causing spoilage/food poisoning | ++ | ++ |
| *Streptococcus pneumoniae* | Bacteria causing pneumonia | ++ | +++ |

| | | | |
|---|---|---|---|
| -: Without antimicrobial activity, +: With antimicrobial activity, ++: With strong antimicrobial activity, +++: With very strong antimicrobial activity | | | |

It was found that the culture supernatant of Lactobacillus sp. SC-2001 had antimicrobial activity against various types of bacteria. The culture supernatant of Lactobacillus sp. SC-2001 exhibited an antimicrobial spectrum that was similar to that of nisin A, which is a known antimicrobial peptide.

### [Experiment 2. Isolation and Purification of Antimicrobial Component of Lactobacillus sp. SC-2001]

### (1) Purification of Active Component

An antimicrobial component contained in culture supernatant of Lactobacillus sp. SC-2001 was isolated and purified by the following method. Lactobacillus sp. SC-2001 was cultured in MRS Broth, and then the culture liquid was centrifuged at 6000 g for 10 minutes to collect the culture supernatant. The resulting culture supernatant was further passed through a cellulose acetate membrane with a pore size of 0.22 µm for cell removal. To 500 mL of the culture supernatant from which cells were removed, 80% ammonium sulfate was added, and the resultant was stirred overnight at 6°C to precipitate protein containing an antimicrobial peptide. The precipitate thus obtained was centrifuged at 13000 g for 60 minutes for collection, and redissolved in 50 mL of a 50-mM sodium phosphate buffer (pH5.6, hereinafter also called "buffer A"). The resulting solution was loaded on a Celite column (manufactured by biotage) for removal of impurities. Subsequently, buffer exchange was carried out with the use of an Amicon Ultra membrane 3 kDa (manufactured by Merck Millipore Corporation) that had been equilibrated with buffer A. Further, with the use of AKTA pure Fraction Collector (manufactured by Cytiva) to which Hitrap SP FF 1 mL (manufactured by GE Healthcare) was connected, fractionation was carried out. Regarding the mobile phase, buffer A was used as a mobile phase A, a 50-mM sodium phosphate buffer (pH5.6) containing 1-M NaCl was used as a mobile phase B, to carry out cation-exchange chromatography at a flow speed of 1 mL/min. Gradient conditions were set at an initial mobile phase composition of B 0% and a final composition of B 100%, and the gradient time was set at 10 minutes. Fractions were collected each in an amount of 1 mL. Among these, an active fraction was further purified with the use of AKTA pure Fraction Collecter (manufactured by Cytiva) to which 3 mL RESOURCE PRC (manufactured by GE Healthcare) was connected. Regarding the mobile phase, 0.1% formic acid was used as a mobile phase A and 0.1% formic acid and ethanol were used as a mobile phase B, to carry out reverse-phase chromatography at a flow speed of 1 mL/min. Gradient conditions were set at an initial mobile phase composition of B 0% and a final composition of B 100%, and the gradient time was set at 10 minutes. Fractions were collected each in an amount of 1 mL. The purified fraction was stored at -30°C. The antimicrobial activity of the fraction thus obtained was evaluated in the following manner.

### (2) Antimicrobial Activity Test

Like in Experiment 1A, antimicrobial activity of the antimicrobial peptide was evaluated by a spot-on-lawn method. As the test bacteria, Lactobacillus sakei (NBRC15893) was used. After overnight culturing, the test bacteria were mixed and diluted with Lactobacilli AOAC agar so as to achieve a density of 4×10⁷ CFU/mL, and the resultant was spread over an MRS plate containing 2% agar. The test liquid was spotted on the test bacteria, and incubated overnight at 30°C. The size of the growth inhibition circle of L. sakei was evaluated as the antimicrobial activity.

### [Experiment 3. Structure Determination of Antimicrobial Substance]

### (1) Acidic Trypsin Digestion

By the method described below, an antimicrobial polypeptide contained in culture supernatant of Lactobacillus sp. SC-2001 was identified. Firstly, 250 µL of the purified active fraction was separated by Tris-Tricin SDS Page, and then a target band was cut out, from which protein was extracted with the use of Protein Extraction Kit from Gel Slices (manufactured by Cosmo Bio). The sample containing the protein was subjected to trypsin digestion with the use of AccuMAP (trademark) Low pH Protein Digestion Kits (manufactured by promega). The sample was reduced/alkylated according to the protocol of the Digestion Kits, followed by addition of 25 µL of rLys-C and reaction at 37°C for 4 hours. The resulting reaction solution was diluted, to which 25 µL of rLys-C was further added, and the resultant was left for reaction at 37°C for 16 hours. Subsequently, 20 µL of Trypsin was added, incubation was carried out at 37°C for 3 hours, and 20% trifluoroacetic acid (TFA) was added to stop the reaction. After desalination of the digestion product, development of the reaction was checked with the use of a mass spectrometer.

### (2) Chemical Fragmentation with Cyanogen Bromide

The purified active fraction in an amount of 100 µL was dissolved in 70% (v/v) formic acid containing 50-µmol cyanogen bromide. The reaction was allowed to proceed at room temperature for 18 hours. The details of the method are as described by E Gross, Methods in enzymology, 27, 238(1967). After desalination, the fragmented peptides were subjected to analysis on a mass spectrometer.

### (3) Mass Spectrometry

Analysis of the peptide fragments in the active fraction was carried out by liquid chromatography-tandem mass spectrometry (LC/MS/MS) in which electrospray ionization was used for interface. As the liquid chromatograph and autosampler, Ultimate 3000 RSLCnano (manufactured by Thermo Fisher Scientific) was used. Formic acid (0.5%) was used as a mobile phase A and formic acid (0.5%) and methanol were used as a mobile phase B, to carry out measurement at a flow speed of 250 nL/min. Gradient conditions were set at an initial mobile phase composition of B 5% and a final composition of B 55%, and the gradient time was set at 90 minutes. As the column for analysis, Acclaim PepMap RSLC 75 µm × 50 cm nano Viper C18, 2 µm, 100 Å (manufactured by Thermo Fisher Scientific) was used. As the mass spectrometer, Q Exactive HF (manufactured by Thermo Fisher Scientific) was used, and the MS/MS spectra were acquired in the Data Dependent Acquisition (DDA) mode. As the solvent for measurement, 100% methanol and 0.1% trifluoroacetic acid were used.

### (4) De Novo Sequencing

The product ion spectra thus obtained were subjected to sequence analysis of the antimicrobial peptide with the use of PEAKS Studio 10.6.

### (5) Comparison of Predicted Sequence with Genomic Analysis

The sequence thus predicted by the de novo sequencing was searched for in the six-frame translation results of the total genome base sequence of Lactobacillus sp. SC-2001 strain.

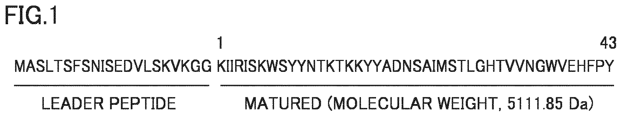

In the above-described manner, the amino acid sequence of the antimicrobial peptide contained in the purified fraction was determined. The precursor full-length sequence of the antimicrobial peptide is given as SEQ ID NO: 1, and the genome sequence thereof is given as SEQ ID NO: 2. After translation, the polypeptide having the amino acid sequence as set forth in SEQ ID NO: 1 is cleaved by peptidase and the leader peptide is cut off, to become a mature antimicrobial peptide having the amino acid sequence as set forth in SEQ ID NO: 3 (Fig. 1).

### [Experiment 4. Homology Search on Amino Acid Sequence of Antimicrobial Peptide]

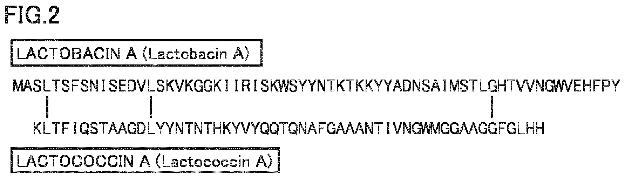

The amino acid sequence of the antimicrobial peptide thus identified was subjected to homology search with the amino acid sequences of known antimicrobial peptides registered in DRAMP (Kang, X. et al., Sci. Data 6, 148(2019)) and BACTIBASE (Hammami, R. et al., BMC Microbiol., 10, 22(2010)), each of which is an antimicrobial peptide database. As a result, the peptide having the highest homology with the antimicrobial peptide contained in the purified fraction was lactococcin A (SEQ ID NO: 8), but there was little coincidence (Fig. 2).

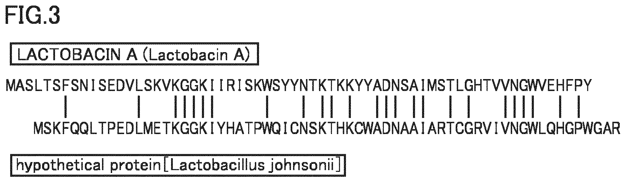

By BLAST, with the amino acid sequence of the polypeptide including the leader sequence entered in Query, and with "All non-redundant GenBank CDS translations + PDB + SwissProt + PIR + PRF excluding environmental samples from WGS projects" selected for Database, homology search was carried out. As a result, it was found that the peptide with the highest homology was a hypothetical protein having the sequence "MSKFQQLTPEDLMETKGGKIYHATPWQICNSKTHKCWADNAAIARTCGRVIV NGWLQHGPW" (SEQ ID NO: 9) (derived from Lactobacillus johnsonii), but the coincidence rate was half or less (Fig. 3). From the above, it is conceivable that the polypeptide as set forth in SEQ ID NO: 1 that was found in Lactobacillus sp. SC-2001 was a novel antimicrobial peptide.

### [Experiment 5. Antimicrobial Activity Test on Synthesized Peptide]

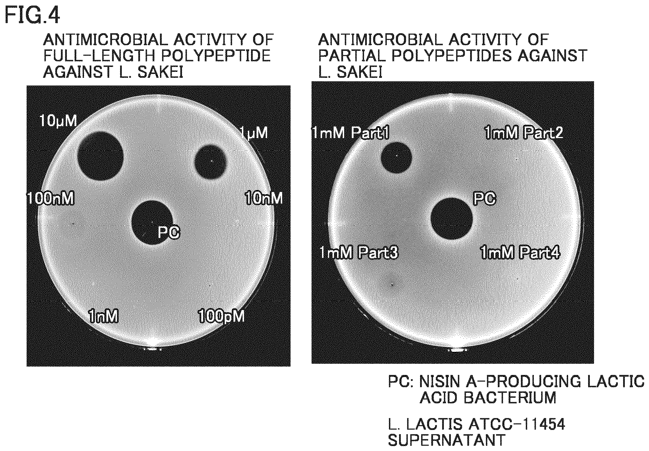

For the mature full-length lactobacin A consisting of the amino acid sequence as set forth in SEQ ID NO: 3 and partial polypeptides consisting of the amino acid sequences as set forth in SEQ ID NOs: 4 to 7 (Table 3), antimicrobial activity was evaluated. Each polypeptide was dissolved in water to achieve the concentration specified in Table 4 or Table 5. The polypeptides were chemically synthesized peptides obtained from Hokkaido System Science Co., Ltd.. By the same spot-on-lawn method as adopted in the above-mentioned antimicrobial activity test, the polypeptide was added in drops to form a growth inhibition circle of L. sakei, and thereby the antimicrobial activity was evaluated. As a positive control, culture supernatant of a nisin A-producing lactic acid bacterium (Lactococcus lactis ATCC-11454 strain) was used. As a control polylysine, ε-Poly-L-lysine coating solution, Code No. SPL01 (manufactured by Cosmo Bio Co., Ltd.) was used. The molecular weight was assumed as 2500, and the polylysine product (about 50 µM) in a concentration of 0.0125% was dried under reduced pressure and redissolved in water the volume of which was for achieving 20-fold concentration (about 1 mM, 2.5 mg/mL), 40-fold concentration (about 2 mM, 5.0 mg/mL), and 80-fold concentration (about 4 mM, 10 mg/mL), respectively, before subjected to antimicrobial activity testing. Results of the antimicrobial activity test of the synthesized polypeptides are given in Table 4, Table 5, and Fig. 4.

**[Table 3]**

| | | |
|---|---|---|
| Full-length sequence (1-43 aa) | SEQ ID NO: 3 | KIIRISKWSYYNTKTKKYYADNSAIMSTLGHTVVNGWVEHFPY |
| part 1 (1-17 aa) | SEQ ID NO: 4 | K11R1SKWSYYNTKTKK |
| part 2 (18-43 aa) | SEQ ID NO: 5 | YYADNSA1MSTLGHTVVNGWVEHFPY |
| part 3 (1-9 aa) | SEQ ID NO: 6 | KIIRISKWS |
| part 4 (10-43 aa) | SEQ ID NO: 7 | YYNTKTKKYYADNSAIMSTLGHTVVNGWVEHFPY |

**[Table 4]**

| | Mature full-length peptide | | | | | | Nisin A-producing strain supernatant |
|---|---|---|---|---|---|---|---|
| | 10 µM | 1 µM | 100 nM | 10 nM | 1 nM | 100 pM | |
| Antimicrobial activity | ++ | ++ | + | - | - | - | ++ |

| | | | | | | | |
|---|---|---|---|---|---|---|---|
| -: Without antimicrobial activity, +: With antimicrobial activity, ++: With strong antimicrobial activity | | | | | | | |

**[Table 5]**

| | Partial peptide (1 mM) | | | | Polylysine | | | | Nisin A-producing strain supernatant |
|---|---|---|---|---|---|---|---|---|---|
| | part 1 | part 2 | part 3 | part 4 | 0.125 mg/mL | 2.5 mg/mL | 5.0 mg/mL | 10 mg/mL | |
| Antimicrobi al activity | ++ | - | + | - | - | ++ | ++ | ++ | ++ |

| | | | | | | | | | |
|---|---|---|---|---|---|---|---|---|---|
| -: Without antimicrobial activity, +: With antimicrobial activity, ++: With strong antimicrobial activity | | | | | | | | | |

The synthesized mature full-length polypeptide at a concentration equal to or more than 100 nM was found to have antimicrobial activity against L. sakei. It was also found that the partial polypeptides having the N-terminal sequence of lactobacin A (part 1 and part 3) had antimicrobial activity against L. sakei. It was also found that part 1 had antimicrobial activity at a similar level to that of polylysine.

### [Experiment 6. Wide pH-Range Stability Test]

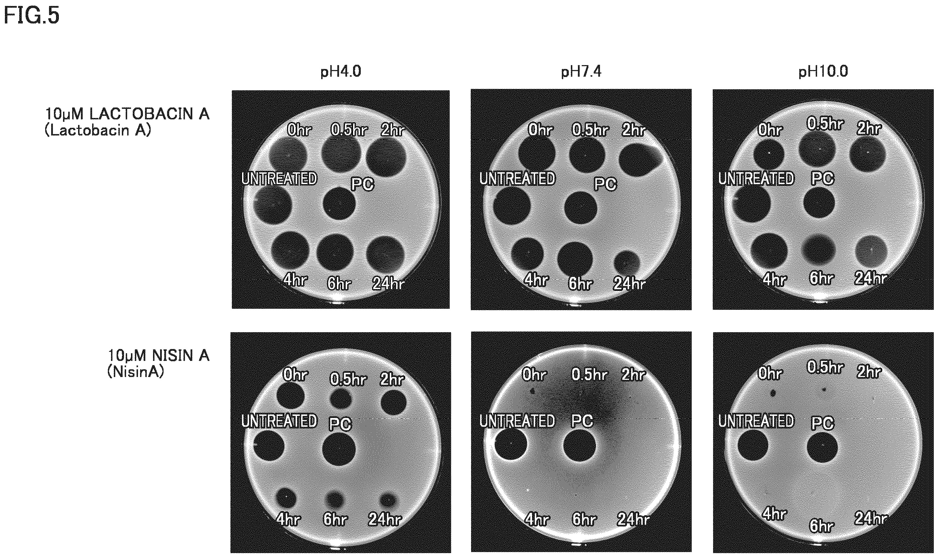

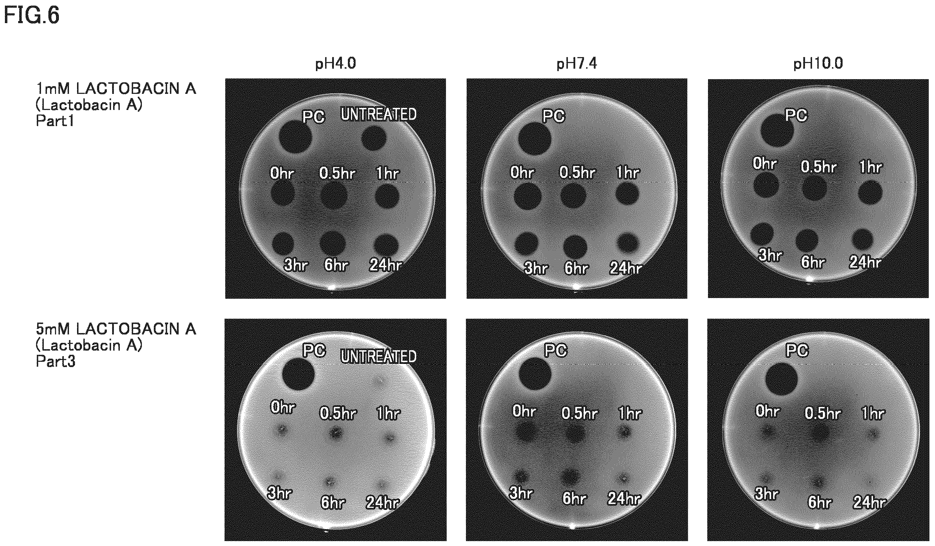

For 10-µM lactobacin A, 10-µM nisin A, 1-mM part 1, and 5-mM part 3, evaluation was carried out to see if they had antimicrobial activity over a wide range of pH. Each peptide was redissolved and prepared in the following buffer; Sodium acetate-acetic acid (50 mM, pH4.0), Sodium phosphate (50 mM, pH7.4), glycine-NaOH (50 mM, pH10.0). The test solution thus prepared at the above-specified pH was incubated at 30°C. The duration of the incubation was 0 hours, 0.5 hours, 2 hours, 4 hours, 6 hours, and 24 hours, and at the completion of the incubation, TFA was added in a final concentration of 0.2%. A pH test paper was used to make sure that the test solution after TFA addition had an acidic pH. By the same spot-on-lawn method as adopted in the above-mentioned antimicrobial activity test, the polypeptide was added in drops to form a growth inhibition circle of L. sakei, and thereby the antimicrobial activity of the polypeptide after pH treatment was evaluated. As a positive control (PC), culture supernatant of a nisin A-producing lactic acid bacterium (Lactococcus lactis ATCC-11454 strain) was used. A sample that was prepared solely with a 0.2% TFA aqueous solution was used as an untreated control. Results are given in Table 6, Fig. 5, and Fig. 6.

Nisin A used in Examples was Nisin from Lactococcus lactis 2.5% (manufactured by Sigma-Aldrich corporation) that was used after concentration and purification. The details of nisin A are as described by J. C. Slootweg et al., J. Pept. Sci., 19, 692-699(2013). To 1 g of commercially available 2.5% nisin A (containing about 25 mg of nisin A), 25 mL of water was added, and the resultant was stirred for 15 minutes at room temperature. Centrifugation (15 minutes, 2500 rpm) was carried out to separate off the precipitate, and only the supernatant was transferred to another tube. To the resulting solution, 20 mL of dichloromethane was added. To collect the white precipitate that was present between the aqueous layer and the organic layer, the upper layer and the lower layer were removed. Another round of centrifugation (15 minutes, 2500 rpm) was carried out to remove, again, the upper layer and the lower layer. The intermediate layer that was sought after was dried in a reduced-pressure centrifuge and re-dissolved in 7.45 mL of water, and thereby a 1-mM purified Nisin product was obtained. The resulting purified product was used as nisin A in each stability test.

**[Table 6]**

| | | 0 hr | 0.5 hr | 2 hr | 4 hr | 6 hr | 24 hr | Untreated |
|---|---|---|---|---|---|---|---|---|
| Lactobacin A (10 µM) | pH 4.0 | ++ | ++ | ++ | ++ | ++ | ++ | ++ |
| | pH 7.4 | ++ | ++ | ++ | ++ | ++ | ++ | |
| | pH 10.0 | ++ | ++ | ++ | ++ | ++ | ++ | |
| Nisin A (10 µM) | pH 4.0 | ++ | ++ | ++ | + | + | + | ++ |
| | pH 7.4 | - | - | - | - | - | - | |
| | pH 10.0 | - | + | - | - | - | - | |
| Lactobacin A part 1 (1 mM) | pH 4.0 | ++ | ++ | ++ | ++ | ++ | ++ | ++ |
| | pH 7.4 | ++ | ++ | ++ | ++ | ++ | ++ | |
| | pH 10.0 | ++ | ++ | ++ | ++ | ++ | ++ | |
| Lactobacin A part 3 (5 mM) | pH 4.0 | + | + | + | + | + | + | + |
| | pH 7.4 | + | + | + | + | + | + | |
| | pH 10.0 | + | + | + | + | + | + | |

| | | | | | | | | |
|---|---|---|---|---|---|---|---|---|
| -: Without antimicrobial activity, +: With antimicrobial activity, ++: With strong antimicrobial activity | | | | | | | | |

Lactobacin A did not lose its antimicrobial activity after 24 hours of incubation in a wide pH range of pH4 to pH10.0. On the other hand, nisin A lost its antimicrobial activity in the neutral range and the alkaline range. The partial polypeptides of lactobacin A also did not degrade in their activity under low-pH and high-pH conditions. It was demonstrated that lactobacin A and the partial polypeptides thereof had antimicrobial activity within a wide range of pH and were excellent in pH stability.

### [Experiment 7. Thermal Stability Test]

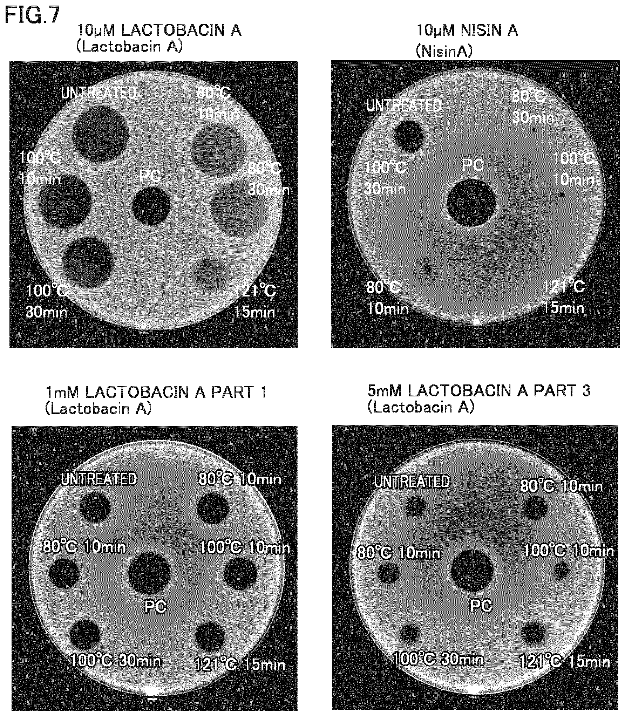

For 10-µM lactobacin A, 10-µM nisin A, 1-mM part 1, and 5-mM part 3, evaluation was carried out to see if they had antimicrobial activity after heat treatment. The test solution was subjected to heat treatment at 80°C for 10 minutes, or at 80°C for 30 minutes, or at 100°C for 10 minutes, or at 100°C for 30 minutes, or at 121°C for 15 minutes. By the same spot-on-lawn method as adopted in the above-mentioned antimicrobial activity test, the polypeptide was added in drops to form a growth inhibition circle of L. sakei, and thereby the antimicrobial activity of the polypeptide after heat treatment was evaluated. As a positive control (PC), culture supernatant of a nisin A-producing lactic acid bacterium (Lactococcus lactis ATCC-1 1454 strain) was used. A sample without the heat process was used as an untreated control. Results are given in Table 7 and Fig. 7.

**[Table 7]**

| | 80°C 10 mm | 80°C 30 min | 100°C 10 min | 100°C 30 min | 121°C 15 min | Untreated |
|---|---|---|---|---|---|---|
| Lactobacin A (10 µM) | ++ | ++ | ++ | ++ | + | ++ |
| Nisin A (10 µM) | + | - | - | - | - | ++ |
| Lactobacin A part 1 (1 mM) | ++ | ++ | ++ | ++ | ++ | ++ |
| Lactobacin A part 3 (5 mM) | + | + | + | + | + | + |

| | | | | | | |
|---|---|---|---|---|---|---|
| -: Without antimicrobial activity, +: With antimicrobial activity, ++: With strong antimicrobial activity | | | | | | |

Lactobacin A did not lose its antimicrobial activity after heat treatment at 80°C or 100°C for 10 minutes or 30 minutes, or at 121°C for 15 minutes. Nisin A retained its activity after heat treatment at 80°C for 10 minutes, but lost its antimicrobial activity after any heat treatment beyond that. The partial polypeptides of lactobacin A did not lose their antimicrobial activity after heat treatment at 80°C or 100°C for 10 minutes or 30 minutes, or at 121°C for 15 minutes. It was demonstrated that lactobacin A and the partial polypeptides thereof had excellent thermal stability.

### [Experiment 8. Oxidation Stability Test]

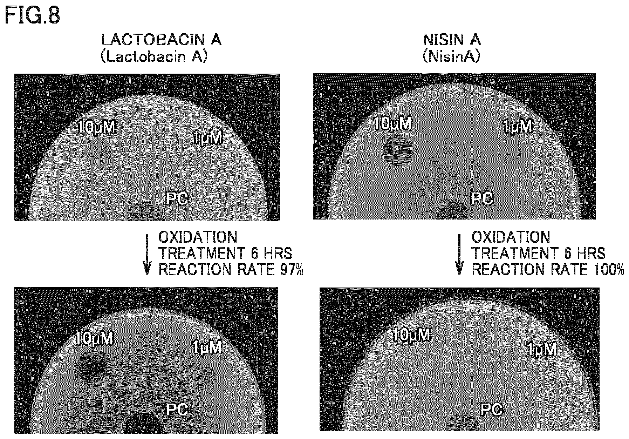

For lactobacin A and nisin A, evaluation was carried out to see if they had antimicrobial activity after oxidation treatment. The method of oxidation treatment is as described by Toshihiro T. et al., J. Proteome Res., 12, 753-762(2013). To a 1-mM solution of lactobacin A or nisin A, hydrogen peroxide was added to achieve a final concentration of 10 mM, followed by reaction at 40°C for 6 hours. After the completion of reaction, the reaction liquid was diluted with water to achieve a polypeptide concentration of 10 µM or 1 µM. By the same spot-on-lawn method as adopted in the above-mentioned antimicrobial activity test, the polypeptide was added in drops to form a growth inhibition circle of L. sakei, and thereby the antimicrobial activity of the polypeptide after oxidation treatment was evaluated. Development of the oxidation reaction was calculated as a relative value of the peak area obtained by LC/MS measurement of the oxidation-treated test solution relative to the non- oxidation-treated test solution. As a positive control (PC), culture supernatant of a nisin A-producing lactic acid bacterium (Lactococcus lactis ATCC-11454 strain) was used. Results are given in Table 8 and Fig. 8.

**[Table 8]**

| | Lactobacin A | | Nisin A | |
|---|---|---|---|---|
| | 10 µM | 1 µM | 10 µM | 1 µM |
| Before oxidation treatment | ++ | + | ++ | + |
| After oxidation treatment | ++ | + | - | - |

| | | | | |
|---|---|---|---|---|
| -: Without antimicrobial activity, +: With antimicrobial activity, ++: With strong antimicrobial activity | | | | |

Lactobacin A did not lose its antimicrobial activity after oxidation treatment with 10-mM hydrogen peroxide. On the other hand, nisin A lost its antimicrobial activity after the same oxidation treatment. It was demonstrated that lactobacin A had excellent oxidation stability.

## Claims

1. A polypeptide with antimicrobial activity, comprising any one of the following amino acid sequences:
(1a) an amino acid sequence as set forth in SEQ ID NO: 3;
(1b) an amino acid sequence with an identity of 80% or more to the amino acid sequence as set forth in SEQ ID NO: 3;
(1c) the amino acid sequence as set forth in SEQ ID NO: 3 with deletion, substitution, insertion, and/or addition of one or several amino acid residues;
(1d) the amino acid sequence of any one of (1a) to (1c) with deletion of 1 to 26 amino acid residues from a C terminus thereof;
(2a) an amino acid sequence as set forth in SEQ ID NO: 4;
(2b) an amino acid sequence with an identity of 80% or more to the amino acid sequence as set forth in SEQ ID NO: 4;
(2c) the amino acid sequence as set forth in SEQ ID NO: 4 with deletion, substitution, insertion, and/or addition of one or several amino acid residues;
(2d) the amino acid sequence of any one of (2a) to (2c) with deletion of 1 to 8 amino acid residues from a C terminus thereof;
(3a) an amino acid sequence as set forth in SEQ ID NO: 6;
(3b) an amino acid sequence with an identity of 80% or more to the amino acid sequence as set forth in SEQ ID NO: 6; and
(3c) the amino acid sequence as set forth in SEQ ID NO: 6 with deletion, substitution, insertion, and/or addition of one or several amino acid residues.

2. A polypeptide with antimicrobial activity, comprising at least a partial amino acid sequence of the amino acid sequence of any one of (1a) to (1c) according to claim 1.

3. A precursor polypeptide for the polypeptide according to claim 1 or 2.

4. The polypeptide according to claim 3, having an amino acid sequence as set forth in SEQ ID NO: 1.

5. A nucleic acid coding for the polypeptide according to any one of claims 1 to 4.

6. The nucleic acid according to claim 5, having a DNA sequence as set forth in SEQ ID NO: 2.

7. A vector comprising the nucleic acid according to claim 5 or 6.

8. A transformant in which the nucleic acid according to claim 5 or 6 is introduced.

9. A method of producing a polypeptide, comprising using the transformant according to claim 8.

10. A composition comprising the polypeptide according to claim 1 or 2 and an additive.
